# EUROPEAN PATENT APPLICATION

(11) **EP 3 437 679 A1**
(43) Date of publication of application: **06.02.2019**
(21) Application number: 17775171.6
(22) Date of filing: 29.03.2017
(51) Int. Cl.: A61M 5/28

(54) **SEAL MEMBER, SYRINGE ASSEMBLY, AND PREFILLED SYRINGE**

(30) Priority: 30.03.2016 JP 2016067698
(71) Applicant: Terumo Kabushiki Kaisha, Tokyo 151-0072 (JP)
(72) Inventor: TACHIKAWA Kouichi, Nakakoma-gun Yamanashi 409-3853 (JP); OSADA Yasuhisa, Nakakoma-gun Yamanashi 409-3853 (JP)
(74) Representative: Casalonga
(86) International application number: PCT/JP2017/012794
(87) International publication number: WO 2017/170634

(57) **Abstract**

Provided is a seal member, comprising a main body part, a closed end part, and a cleavable part. The main body part further comprises an aperture part which is formed in the proximal end thereof, and a tight contact part which is capable of forming a fluid-tight seal with an inner wall of a cylinder body. The closed end part is formed integrally and continuously with the main body part at the distal end of the main body part, and seals off the distal end of the main body part. The cleavable part is formed in the closed end part, and is connected with the aperture part. The cleavable part cleaves by a prescribed pressure being applied in a drug solution storage space, and a liquid transmission aperture which connects with the aperture part is formed in the closed end part.

## Description

### Technical Field

The present invention relates to a syringe assembly including a syringe capable of storing a drug solution beforehand, a seal member that seals drug solution in a syringe in a fluid-tight manner, a syringe assembly including the seal member, and a prefilled syringe.

### Background Art

In recent years, a prefilled syringe in which a drug solution is filled in the syringe beforehand is widely used. This type of prefilled syringe omits necessity to aspirate the drug solution from a vial bottle into the syringe at the time of drug solution administration, making it possible to reduce the time needed for administration.

For example, Patent Literature 1 discloses a technique to prevent a drug solution from leaking out of a syringe before administration of the drug solution. Patent Literature 1 describes a technique of providing a slit to a stopper for sealing the drug solution.

### Citation List

### Patent Literature

Patent Literature 1: JP 8-528629 A

### Summary of Invention

### Technical Problem

With the technique described in Patent Literature 1, however, there is a possibility that the drug solution stored in the syringe may leak from the slit during storage or transportation. Furthermore, in the case of a prefilled syringe in which a drug solution and a medical agent are mixed, there is a possibility that the drug solution leaks out from the medicine chamber containing the drug solution and the drug solution and the medical agent are mixed against an intention of the user.

An object of the present invention is to provide a seal member, a syringe assembly, and a prefilled syringe capable of preventing drug solution from leaking out of a syringe before administration of a drug solution in consideration of the above-described problems.

### Solution to Problem

In order to achieve the above object, a seal member of the present invention is used for a syringe assembly comprising a syringe including a cylinder body that is configured to be filled with a drug solution, and is configured to be a seal member disposed in the cylinder body. In addition, the seal member comprises a tubular main body part, a closed end part, and a cleavable part. The main body part further includes an aperture part which is formed in a proximal end thereof and a tight contact part configured to be form a fluid-tight seal with an inner wall of the cylinder body. The closed end part is formed integrally and continuously with the main body part at the distal end of the main body part, and seals off the distal end of the main body part. The cleavable part is formed in the closed end part, and is connected with the aperture part. In addition, the main body part is disposed in the cylinder body together with the gasket slidable in the cylinder body, and is positioned distal of the gasket in the cylinder body, thereby forming a drug solution storage space for storing the drug solution together with the gasket and the cylinder body. The cleavable part cleaves by a prescribed pressure being applied in a drug solution storage space, and forms a liquid transmission aperture which connects with the aperture part at the closed end part.

The syringe assembly of the present invention includes the above-described seal member and a syringe. The seal member is disposed in the cylinder body.

Furthermore, the prefilled syringe of the present invention comprises the above-described syringe assembly, a gasket, and a drug solution. The gasket is slidably disposed in a cylinder hole of the cylinder body, within a syringe including a cylinder body filled with a drug solution. The drug solution is stored in a drug solution storage space formed by the seal member, the gasket, and the cylinder body.

### Advantageous Effects of Invention

According to the seal member, the syringe assembly and the prefilled syringe of the present invention, it is possible to seal the drug solution in the syringe.

### Brief Description of Drawings

FIG. 1 is a perspective view illustrating a prefilled syringe according to a first exemplary embodiment of the present invention.
FIG. 2 is a cross sectional view illustrating the prefilled syringe according to the first exemplary embodiment of the present invention.
FIG. 3 is a perspective view of a seal member according to the first exemplary embodiment of the present invention as viewed from a distal end side.
FIG. 4 is a perspective view of the seal member according to the first exemplary embodiment of the present invention as viewed from the rear end side.
FIG. 5 is a cross sectional view illustrating the seal member according to the first exemplary embodiment of the present invention.
FIG. 6 is a cross sectional view illustrating the seal member according to the first exemplary embodiment of the present invention.
FIG. 7 is a cross sectional view illustrating administration operation of a drug solution in a prefilled syringe according to the first exemplary embodiment of the present invention.
FIG. 8 is a cross sectional view illustrating administration operation of a drug solution in a prefilled syringe according to the first exemplary embodiment of the present invention.
FIG. 9 is a cross sectional view illustrating administration operation of a drug solution in the prefilled syringe according to the first exemplary embodiment of the present invention.
FIG. 10 is a cross sectional view illustrating administration operation of a drug solution in the prefilled syringe according to the first exemplary embodiment of the present invention.
FIG. 11 is a cross sectional view illustrating administration operation of a drug solution the prefilled syringe according to the first exemplary embodiment of the present invention.
FIG. 12 is a cross sectional view illustrating a state after a drug solution is administered in the prefilled syringe according to the first exemplary embodiment of the present invention.
FIG. 13 is an enlarged cross sectional view illustrating a seal member after administration of a drug solution in the prefilled syringe according to the first exemplary embodiment of the present invention.
FIG. 14 is a cross sectional view illustrating a prefilled syringe according to a second exemplary embodiment of the present invention.
FIG. 15 is a cross sectional view illustrating a state of mixing a drug solution and a medical agent in the prefilled syringe according to the second exemplary embodiment of the present invention.
FIG. 16 is a cross sectional view illustrating a state in which a drug solution and a medical agent are mixed in the prefilled syringe according to the second exemplary embodiment of the present invention.
FIG. 17 is a cross sectional view illustrating a state after administration of a mixed fluid of a drug solution and a medical agent in the prefilled syringe according to the second exemplary embodiment of the present invention.

### Description of Embodiments

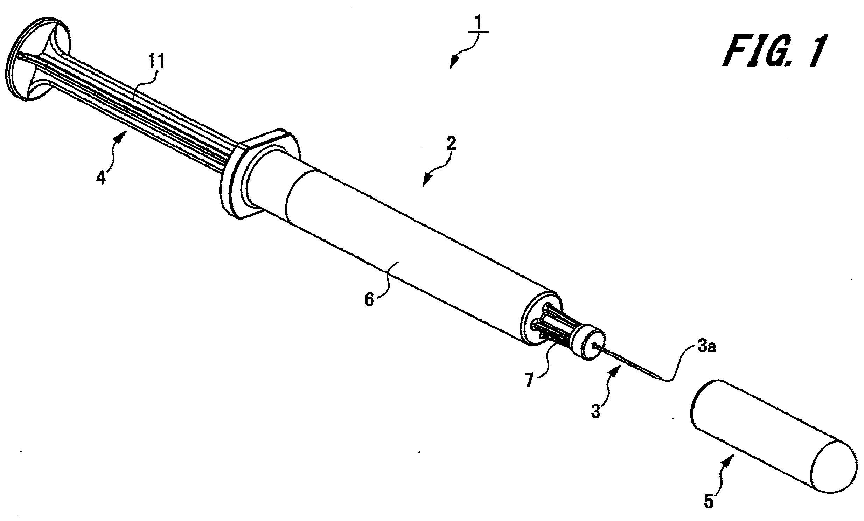

Hereinafter, exemplary embodiments of a seal member, a syringe assembly and a prefilled syringe according to the present invention will be described with reference to FIGS. 1 to 17. In the drawings, common members are denoted by the same reference numerals. Moreover, the present invention is not limited to the following embodiments.

### 1. First Exemplary Embodiment

### 1-1. Configuration example of prefilled syringe

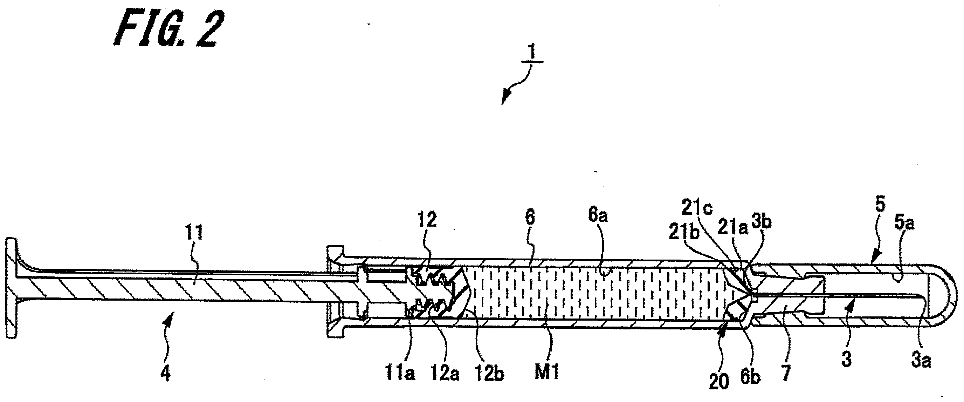

First, an exemplary configuration of a prefilled syringe according to a first exemplary embodiment (hereinafter referred to as "this example") of the present invention will be described with reference to FIGS. 1 and 2.

FIG. 1 is a perspective view illustrating a prefilled syringe. FIG. 2 is a cross sectional view illustrating the prefilled syringe.

The prefilled syringe 1 of this example stores beforehand a drug solution M1 to be administered to a living body. As illustrated in FIGS. 1 and 2, a prefilled syringe 1 includes a syringe 2 that stores a drug solution M1, a needle tube 3 fixed to the syringe 2, a pusher 4, a cap 5, and a seal member 20.

### [Syringe]

The syringe 2 includes a cylinder body 6 in which the drug solution M1 is filled and a fixed part 7 continuous with the cylinder body 6. The cylinder body 6 has a substantially cylindrical shape. The fixed part 7 is continuously formed on the distal end side of the cylinder body 6. The proximal end side of the cylinder body 6 is open. The drug solution M1 is filled in the cylinder hole 6a of the cylinder body 6, and a gasket 12 of the pusher 4 described below is inserted from a proximal end side of the cylinder body 6.

A proximal end part 3b of the needle tube 3 fixed to the fixed part 7 protrudes from an inner distal end surface 6b of the distal end part of the cylinder hole 6a of the cylinder body 6, allowing connection between the needle tube 3 and the cylinder hole 6a. The seal member 20 is disposed on the inner distal end surface 6b of the cylinder hole 6a.

While this example describes a case where the cylinder body 6 of the syringe 2 has a substantially cylindrical shape, the shape of the cylinder body 6 may be a hollow quadrangular prism shape or a hexagonal prism shape.

The fixed part 7 illustrating one example of a holding portion protrudes from the distal end side of the cylinder body 6 in its axial direction. The fixed part 7 has a columnar shape. In the fixed part 7, an outer diameter of an intermediate part in the axial direction of is smaller than an outer diameter of both end parts in the axial direction. This gives the fixed part 7 a shape in which the intermediate part is necked down. The needle tube 3 is fixed to the fixed part 7 by a fixing method such as insert molding, adhesion with an adhesive or the like.

Examples of the constituent material of the syringe 2 having the above-described configuration includes various types of resins such as polyvinyl chloride, polyethylene, polypropylene, cyclic polyolefin, polystyrene, poly- (4-methylpentene-1), polycarbonate, acrylic resin, acrylonitrile-butadiene-styrene copolymers, polyesters such as polyethylene terephthalate, butadiene-styrene copolymers, and polyamides (for example, nylon 6, nylon 6·6, nylon 6·10, and nylon 12). Among them, it is preferable to use a resin such as polypropylene, cyclic polyolefin, polyester, and poly-(4-methylpentene-1). It is preferable that the material of the syringe 2 is substantially transparent in order to ensure the visibility of the interior.

The medical agent to be filled in the syringe 2 may be any medical agent normally used as an injection, and examples of these include protein medical agents such as antibodies, peptide medical agents such as hormones, nucleic acid medical agents, cell medical agents, blood products, vaccines to prevent various types of infectious diseases, anticancer agents, anesthetics, narcotics, antibiotics, steroids, protease inhibitors, carbohydrate, heparin, carbohydrate injection solutions such as glucose, electrolyte correction injection solutions such as sodium chloride and potassium lactate, vitamins, fat emulsions, contrast agents, and stimulants.

### [Needle tube]

It is prescribed to use the needle tube 3 having a size of 10 to 33 gauge (outer diameter: ϕ 3.5 mm to 0.2 mm) according to ISO standard for medical needle tube (ISO 9626: 1991/Amd 1: 2001(E)), preferably a size of 16 to 33 gauge (outer diameter: ϕ1.7 mm to 0.2 mm). The distal end of the needle tube 3 has a blade surface formed to make the needle point 3a to have an acute angle. The living body is punctured with the needle point 3a at the distal end of the needle tube 3.

An example of the material of the needle tube 3 is stainless steel. It is not limited thereto, however, and it is possible to use aluminum, an aluminum alloy, titanium, a titanium alloy, and other metals. As the needle tube 3, not only a straight needle but also a tapered needle having a tapered shape at least in part can be used. As the tapered needle, the proximal end part has a larger diameter than the end part of the needle point 3a, and the intermediate part thereof may have a tapered structure. Furthermore, a sectional shape of the needle tube 3 may be not only a circular shape but also a polygonal shape such as a triangle.

Furthermore, a coating agent formed of a silicone resin, a fluorine-based resin, or the like, is applied to a surface of the needle tube 3 on the needle point 3a side. This makes it possible to reduce friction between the skin and the needle tube when the living body is punctured with the needle tube 3 and to reduce the pain accompanying the puncture.

The needle tube 3 is fixed to the syringe 2 with the needle point 3a protruding outward. Then, the needle point 3a at the distal end of the needle tube 3 protrudes from the distal end of the fixed part 7 of the syringe 2. Moreover, the proximal end part 3b being the proximal end side of the needle tube 3 is exposed from the inner distal end surface 6b of the cylinder body 6 of the syringe 2 toward the cylinder hole 6a. This allows connection between the needle tube 3 and the cylinder hole 6a of the cylinder body 6 of the syringe 2.

### [Pusher]

Next, the pusher 4 will be described.

The pusher 4 includes a push rod 11 and a gasket 12. The push rod 11 is formed in a rod shape and is pressed by a user. The distal end part of the push rod 11 is inserted into the cylinder hole 6a of the cylinder body 6. The length of the push rod 11 in the axial direction is set to be substantially equal to or longer than a length that enables sliding movement of the gasket 12 along the inside of the cylinder hole 6a of the cylinder body 6.

A coupling part 11a is formed at the distal end part of the push rod 11. The coupling part 11a is, for example, a male screw portion formed at the distal end part of the push rod 11. The coupling part 11a is inserted into a coupled part 12a of the gasket 12 and screwed with a coupled part 12a.

The gasket 12 is slidably disposed in the cylinder hole 6a of the cylinder body 6. The gasket 12 is formed in a substantially columnar shape and is in close contact with a wall surface of the cylinder hole 6a of the cylinder body 6 in a fluid-tight manner. The gasket 12 partitions the inside of the cylinder hole 6a of the cylinder body 6 into two. The space on more toward the inner distal end surface 6b side than the gasket 12 in the cylinder hole 6a is to be a fluid chamber in which the drug solution M1 is filled.

The gasket 12 has a closed distal end and the open proximal end. The coupled part 12a is formed at the proximal end of the gasket 12. Furthermore, the distal end part 12b of the gasket 12 is formed in a substantially frustoconical shape corresponding to the shape of the inner distal end surface 6b of the cylinder body 6.

The coupled part 12a is, for example, a female screw portion. The coupled part 12a is screwed with the coupling part 11a of the push rod 11, so as to couple the push rod 11 with the gasket 12. When the push rod 11 is pressed by the user, the gasket 12 slides and moves in the cylinder hole 6a of the cylinder body 6, so as to push the drug solution M1 filled in the cylinder hole 6a toward the proximal end part 3b of the needle tube 3.

While the material of the gasket 12 is not particularly limited, it is preferable to be formed of an elastic material in order to improve fluid tightness with the cylinder body 6. Examples of the elastic material include various rubber materials such as natural rubber, isobutylene rubber and silicone rubber, various thermoplastic elastomers such as olefin type and styrene type, and mixtures of these.

### [Cap]

The cap 5 is formed in a substantially cylindrical shape, with the proximal end open, and the distal end closed. The cap 5 is formed of an elastic member such as rubber or elastomer. As illustrated in FIG. 2, the cap 5 is attached to the distal end side of the syringe 2 so as to cover the needle point 3a of the needle tube 3 and the fixed part 7 of the syringe 2. Then, the needle point 3a side of the needle tube 3 and the fixed part 7 are inserted into the cylinder hole 5a of the cap 5.

Note that the inner diameter of the proximal end part of the cylinder hole 5a of the cap 5 is set to be substantially equal to or slightly smaller than the outer diameter of the intermediate part of the fixed part 7. Therefore, when the cap 5 is attached to the syringe 2, an outer circumferential surface of the intermediate part of the fixed part 7 is brought into close contact with an inner circumferential surface of the cap 5. This allows the needle point 3a side of the needle tube 3, which is the distal end side from the intermediate part of the fixed part 7, to be hermetically sealed by the intermediate part and the inner circumferential surface of the cap 5. As a result, it is possible to prevent bacteria from attaching to a portion of the needle tube 3 including the needle point 3a protruding to the distal end side from the fixed part 7. It is sufficient that the inner circumferential surface of the cap 5 is in close contact with the outer circumferential surface of the fixed part 7 so as to hermetically close a space around the portion protruding from the fixed part 7 of the needle tube 3 to the distal end side.

The inner circumferential surface of the cap 5 tightens, by its elastic force, the necked portion being the intermediate part of the fixed part 7. As a result, the inner circumferential surface of the cap 5 and the necked portion of the fixed part 7 are engaged with each other, making it possible to prevent detachment of the cap 5 from the syringe 2 during conveyance.

Furthermore, the needle point 3a of the needle tube 3 has not punctured the cap 5. This suppresses occurrence of coring in the needle tube 3 and damage in the needle point 3a of the needle tube 3.

### [Seal member]

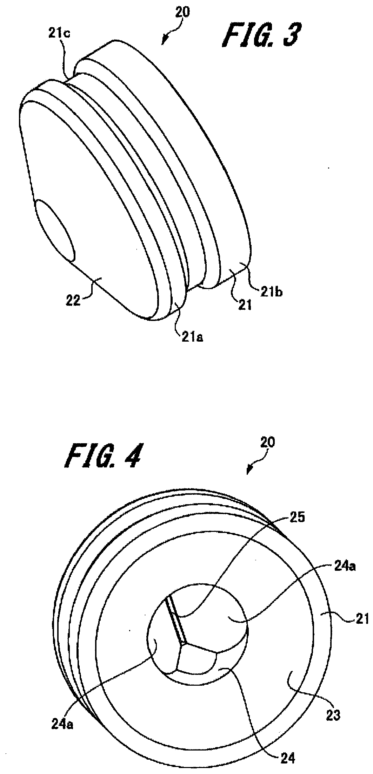

Next, the seal member 20 will be described with reference to FIGS. 3 to 6.

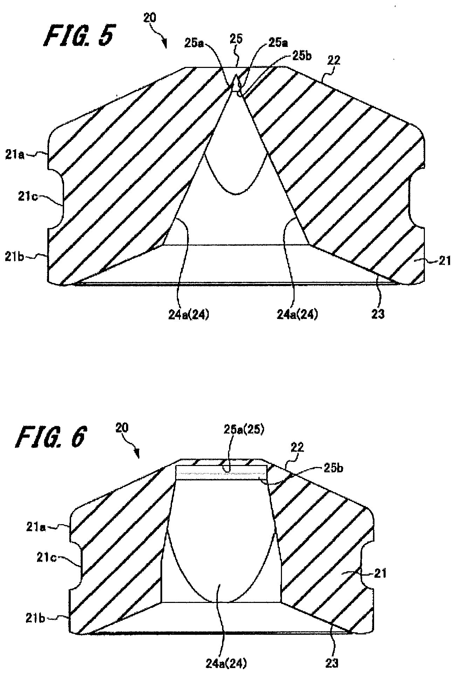

FIGS. 3 and 4 are perspective views illustrating the seal member 20, and FIGS. 5 and 6 are cross sectional views illustrating the seal member 20.

As illustrated in FIGS. 3 and 4, the seal member 20 includes a main body part 21 and a closed end part 22. The main body part 21 has a substantially cylindrical shape. In addition, the main body part 21 is located closer to the distal end side within the cylinder body 6 than the gasket 12, when disposed within the cylinder body 6. Then, a drug solution storage space for storing the drug solution M1 is formed by the main body part 21, the gasket 12, and the cylinder body 6. Furthermore, a closed end part 22 is integrally and continuously formed with the main body part 21, at the distal end of the main body part 21. The closed end part 22 seals the distal end of the main body part 21. The distal end surface of the closed end part 22 is formed in a substantially frustoconical shape complementarily to the shape of the inner distal end surface 6b of the cylinder body 6 in the syringe 2.

As illustrated in FIG. 3, the main body part 21 includes a distal end side rib part 21a as a tight contact part and a proximal end side rib part 21b. The distal end side rib part 21a and the proximal end side rib part 21b are ring-shaped protrusions formed on the outer circumferential surface of the main body part 21. The distal end side rib part 21a and the proximal end side rib part 21b protrude outward in a radial direction from the outer circumferential surface of the main body part 21. The distal end side rib part 21a is formed on the distal end side of the main body part 21, while the proximal end side rib part 21b is formed on the proximal end side of the main body part 21. In addition, a prescribed interval is formed between the distal end side rib part 21a and the proximal end side rib part 21b. Furthermore, the distal end side rib part 21a is adjacent to the inner distal end surface 6b of the cylinder body 6.

The distal end side rib part 21a and the proximal end side rib part 21b are in close contact with a wall surface of the cylinder hole 6a of the cylinder body 6 in a fluid-tight manner. Therefore, the distal end side rib part 21a and the proximal end side rib part 21b form a fluid-tight seal with the inner wall of the cylinder body 6. This configuration achieves fluid-tight sealing of the drug solution M1 filled in the drug solution storage space formed between the seal member 20 and the gasket 12, making it possible to preventing leakage of the drug solution M1 from the seal member 20 and the needle tube 3 in a state before administration of the drug solution. Furthermore, with the two ribs, namely, the distal end side rib part 21a and the proximal end side rib part 21b provided in the main body part 21, it is possible to reliably prevent leakage of the drug solution M1 from the seal member 20.

The distal end side rib part 21a and the proximal end side rib part 21b come in slidable contact with the wall surface of the cylinder hole 6a of the cylinder body 6. This facilitates operation of inserting the seal member 20 into the cylinder hole 6a of the cylinder body 6 in assembling the prefilled syringe 1. In addition, it is possible to prevent tilting of the seal member 20 within the cylinder body 6 by the distal end side rib part 21a and the proximal end side rib part 21b.

Furthermore, a separation part 21c is formed between the distal end side rib part 21a and the proximal end side rib part 21b. The separation part 21c is a recess recessed in a ring shape from the outer circumferential surface of the main body part 21. When the seal member 20 is inserted into the cylinder hole 6a of the cylinder body 6, the separation part 21c separates from the wall surface of the cylinder hole 6a. Therefore, a space is formed by the separation part 21c between the distal end side rib part 21a and the proximal end side rib part 21b. With facilitates deformation of the seal member 20 by the pressure from the drug solution M1 while maintaining the sealability.

As illustrated in FIGS. 4, 5 and 6, an aperture part 23 is formed on the proximal end side of the main body part 21. The aperture part 23 is open in a substantially frustoconical shape complementary to the shape of the distal end part 12b of the gasket 12. Furthermore, a pressurizing part 24 is formed continuously on the distal end side of the aperture part 23. Furthermore, a cleavable part 25 provided at the closed end part 22 continues to the distal end of the pressurizing part 24.

The pressurizing part 24 has two pressurizing surfaces 24a and 24a. As illustrated in FIG. 5, the two pressurizing surfaces 24a and 24a are formed in tapered shapes so that an interval between the two pressurizing surfaces 24a and 24a is continuously reduced as approaching the closed end part 22 and the cleavable part 25. Accordingly, the pressurizing part 24 is formed as a tapered recess. In the closed end part 22, the cleavable part 25 is formed continuously with the two pressurizing surfaces 24a, 24a.

The cleavable part 25 is linearly formed in a direction orthogonal to the center in the axial direction of the main body part 21 and the closed end part 22, that is, in a direction orthogonal to the axial direction of the cylinder body 6. The cleavable part 25 connects with the aperture part 23 formed in the main body part 21. In addition, the closed end part 22 is formed to be thinner in the vicinity of the cleavable part 25 than in the other sites.

The cleavable part 25 has a linear part 25b extending from the pressurizing part 24 in the axial direction of the seal member 20, and two pressurized surfaces 25a, 25a. The pressurized surface 25a is formed continuously with the distal end of the linear part 25b. Then, the two pressurized surfaces 25a, 25a are formed in a tapered shape to have an interval between the two pressurized surfaces 25a and 25a narrows as separated from the linear part 25b and toward the distal end of the closed end part 22. This leads to formation of the cleavable part 25 as a tapered recess having a flow path continuously reduced toward the distal end of the closed end part 22.

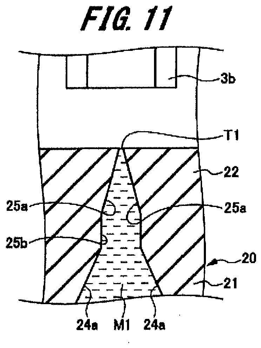

Then, when a prescribed pressure is applied to the drug solution storage space at administration of the drug solution M1 to the living body, the two pressurized surfaces 25a, 25a of the cleavable part 25 deform in a direction away from each other, and this causes cleavage in a thin wall portion in the vicinity of the cleavable part 25 in the closed end part 22, leading to formation of a liquid transmission aperture T1 in the closed end part 22 (refer to FIG. 11). With this configuration, the drug solution M1 stored in the cylinder hole 6a of the cylinder body 6 is discharged to the needle tube 3 via the liquid transmission aperture T1.

While the material of the seal member 20 is not particularly limited, it is preferable to be formed of an elastic material in order to improve fluid tightness with the cylinder body 6. Examples of the elastic material include various rubber materials such as natural rubber, isobutylene rubber and silicone rubber, various thermoplastic elastomers such as olefin type and styrene type, and mixtures of these.

In addition, the syringe 2 and the seal member 20 before filling the drug solution M1 constitute a syringe assembly.

### 1-2. Drug solution administration operation

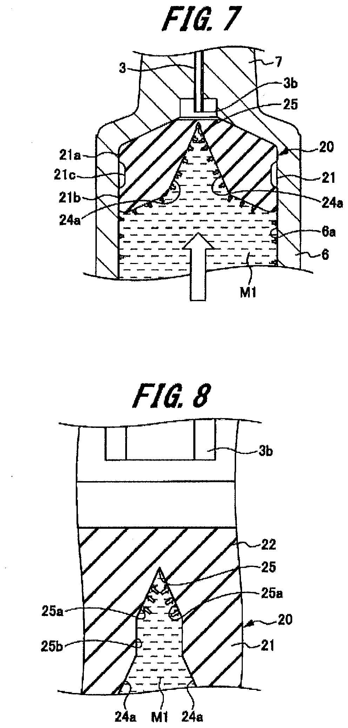

Next, operation of administering the drug solution M1 in the prefilled syringe 1 having the configuration described above will be described with reference to FIGS. 7 to 13.

FIGS. 7 to 13 are explanatory diagrams illustrating drug solution administration operation.

Note that the cap 5 or another member is not punctured with the needle point 3a of the needle tube 3 in a state before administration of the drug solution as illustrated in FIG. 2. This therefore suppresses occurrence of coring in the needle tube 3 and damage in the needle point 3a of the needle tube 3.

First, when the pusher 4 receives pressurizing operation by the user to slidably move the gasket 12 within the cylinder hole 6a, the seal member 20 receives pressure from the drug solution M1 pushed out by the gasket 12 as illustrated in FIG. 7. This allows a force to be applied to the two pressurizing surfaces 24a, 24a of the pressurizing part 24 in directions away from each other due to the pressure from the drug solution M1. Furthermore, as illustrated in FIG. 8, a force is applied to the two pressurized surfaces 25a, 25a of the cleavable part 25 in directions away from each other.

Here, the pressure from the drug solution M1 is received by the two pressurizing surfaces 24a, 24a of the pressurizing part 24 and the two opposed surfaces such as the two pressurized surfaces 25a, 25a of the cleavable part 25, making it possible to integrate pressures from the drug solution M1. Furthermore, with the presence of the linear part 25b provided between the two pressurizing surfaces 24a, 24a and the two pressurized surfaces 25a, 25a, it is possible to further integrate the pressures from the drug solution M1 toward the two pressurized surfaces 25a, 25a of the cleavable part 25 from the pressurizing part 24.

In addition, a ring-shaped separation part 21c is formed on the outer circumferential surface of the main body part 21 in the vicinity of the pressurizing part 24. This separation part 21c facilitates elastic deformation of the seal member 20 by the pressure from the drug solution M1, and facilitates separation of the two pressurizing surfaces 24a, 24a of the pressurizing part 24 away from each other.

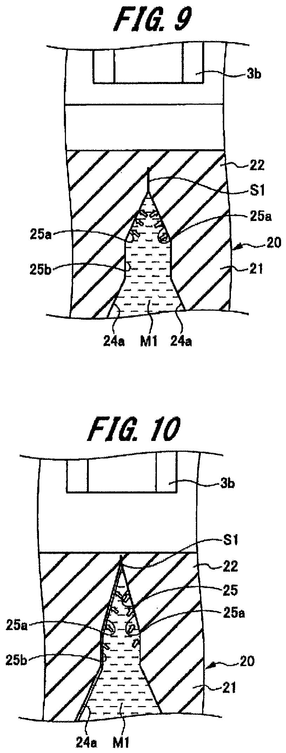

Furthermore, when the drug solution M1 is pushed out toward the inner distal end surface 6b of the cylinder hole 6a by the gasket 12, a force is applied to the two pressurized surfaces 25a, 25a to separate from each other further by the pressure from the drug solution M1 as illustrated in FIG. 9, This configuration forms a slit S1 on each of the distal ends of the two pressurized surfaces 25a, 25a.

When the drug solution M1 is further pushed out toward the inner distal end surface 6b of the cylinder hole 6a by the gasket 12, as illustrated in FIG. 10, the two pressurized surfaces 25a and 25a are further separated away from each other, and the slit S1 is closed end part 22.

The drug solution M1 is further pushed out toward the inner distal end surface 6b of the cylinder hole 6a by the gasket 12, cleaving the distal end of the cleavable part 25 as illustrated in FIG. 11. This forms the liquid transmission aperture T1 connecting with the aperture part 23 allows transmission of the drug solution M1 at a position where the cleavable part 25 is provided at the closed end part 22 of the seal member 20. This allows connection between the cylinder hole 6a of the cylinder body 6 and the needle tube 3 via the aperture part 23 of the seal member 20 and the liquid transmission aperture T1, causing the drug solution M1 to be discharged from the needle tube 3 to be administered to the living body.

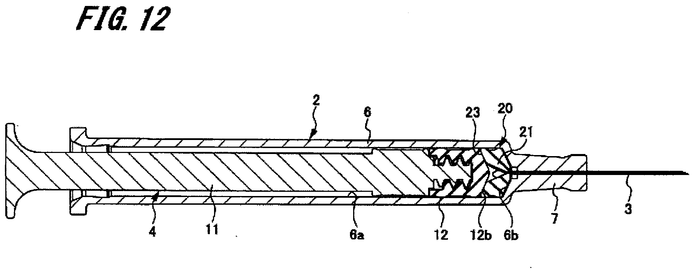

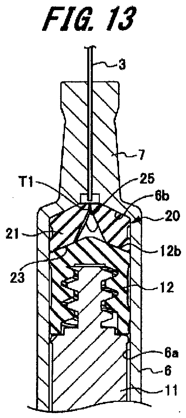

Then, as illustrated in FIGS. 12 and 13, the distal end part 12b of the gasket 12 slides and moves to be inserted into the aperture part 23 of the seal member 20, completing the administration operation of the drug solution M1. Here, the aperture part 23 of the seal member 20 is formed in a substantially frustoconical shape complementary to the shape of the distal end part 12b of the gasket 12. This shape makes it possible to insert the distal end part 12b of the gasket 12 into the aperture part 23 of the seal member 20 without a gap. This makes it possible to reduce the amount of the drug solution M1 remaining between the seal member 20 and the gasket 12.

In addition, the distal end side rib part 21a is provided in the vicinity of the closed end part 22 of the main body part 21, with the distal end side rib part 21a adjacent to the inner distal end surface 6b of the cylinder body 6. This prevents entrance of the drug solution M1 into a portion between the distal end side rib part 21a and the proximal end side rib part 21b from a gap between the inner distal end surface 6b and the closed end part 22 of the seal member 20 when the drug solution M1 is discharged.

In addition, the closed end part 22 of the seal member 20 is formed in a substantially frustoconical shape complementarily to the shape of the inner distal end surface 6b of the cylinder body 6 in the syringe 2. This makes it possible to reduce the amount of the drug solution M1 remaining between the inner distal end surface 6b and the closed end part 22 of the seal member 20 when the drug solution M1 is discharged.

Furthermore, in the seal member 20 of this example, the cleavable part 25 to be cleaved is formed linearly with one cleaving direction. This configuration makes it possible to integrate the stress applied to the cleavable part 25 by the pressure from the drug solution M1, enabling reduction of the resistance generated when the cleavable part 25 is cleaved. This leads to facilitation of cleaving of the cleavable part 25 at the closed end part 22 of the seal member 20 when the drug solution M1 is administered, enabling facilitation of administration operation of the drug solution M1.

### 2. Second Exemplary Embodiment

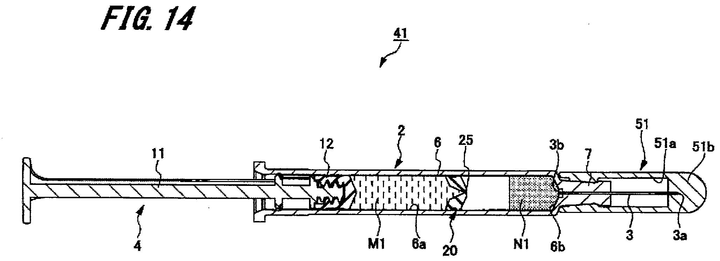

Next, a prefilled syringe according to a second exemplary embodiment will be described with reference to FIG. 14 to FIG. 17.

FIG. 14 is a cross sectional view illustrating a prefilled syringe.

In a prefilled syringe 41 according to the second exemplary embodiment illustrated in FIG. 14, a drug solution M1 and a powdery medical agent N1 are accommodated in the cylinder hole 6a of the cylinder body 6 of the syringe 2. The difference between the prefilled syringe 41 according to the second exemplary embodiment and the prefilled syringe 1 according to the first exemplary embodiment is where the seal member 20 is arranged. Accordingly, the same reference numerals are given to portions common to the prefilled syringe 1 according to the first exemplary embodiment, and redundant explanations are omitted.

The prefilled syringe 41 illustrated in FIG. 14 mixes the drug solution M1 with the medical agent N1, and administers the mixed drug solution M1 and the medical agent N1 to the living body. The seal member 20 is disposed in the cylinder hole 6a of the cylinder body 6 of the syringe 2. In addition, the seal member 20 is arranged slidably in an intermediate part of the cylinder hole 6a in the axial direction.

A space formed by the seal member 20 and the gasket 12 of the pusher 4 in the cylinder hole 6a is filled with the drug solution M1. A medical agent storage space is formed by the inner wall and the inner distal end surface 6b of the cylinder body 6 and the seal site 20. The medical agent storage space stores the powdery medical agent N1. In a state of mixing the drug solution M1 and the medical agent N1, the drug solution M1 is sealed in a fluid-tight manner in the cylinder hole 6a by the seal member 20 and the gasket 12. This makes it possible to prevent the drug solution M1 from leaking out before mixing the drug solution M1 with the medical agent N1, and to prevent mixing of the drug solution M1 and the medical agent N1 against an intention of the user.

A cap 51 is attached to the fixed part 7 of the syringe 2. The cap 51 is formed in a substantially cylindrical shape, with the proximal end side open, and the distal end side closed. A sealing portion 51b is provided on the distal end side in the cylinder hole 51a of the cap 51. Then, the needle point 3a of the needle tube 3 and the fixed part 7 are inserted into the cylinder hole 51a of the cap 51, the sealing portion 51b is punctured with the needle point 3a of the needle tube 3. This makes it possible to prevent leakage of the medical agent N1 from the needle tube 3.

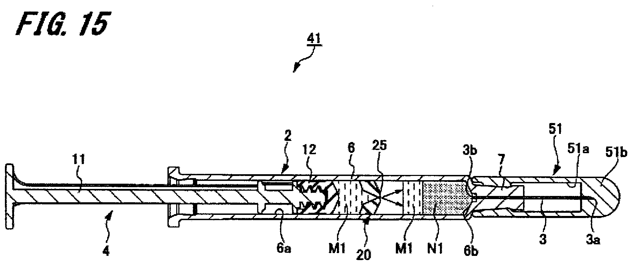

Next, administration operation of the prefilled syringe 41 according to the second exemplary embodiment will be described with reference to FIGS. 15 to 17.

FIGS. 15 to 17 are cross sectional views illustrating administration operation of the prefilled syringe.

First, as illustrated in FIG. 15, the user presses a push rod 11 of the pusher 4 to slide and move the gasket 12 toward the inner distal end surface 6b. This allows the pressure of the drug solution M1 to be applied to the seal member 20 by the gasket 12, cleaving the cleavable part 25 of the seal member 20. This results in achieving connection between the space storing the drug solution M1 and the space storing the medical agent N1 via the liquid transmission aperture T1 formed in the cleavable part 25 of the seal member 20.

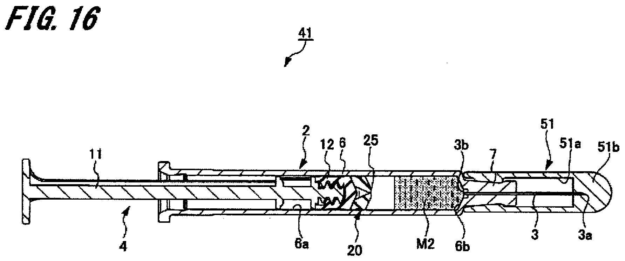

Next, as illustrated in FIG. 16, the push rod 11 is further pushed and the gasket 12 is slidably moved toward the inner distal end surface 6b, coming into contact with the seal member 20 of the gasket 12. Then, the drug solution M1 is pushed out by the gasket 12 to pass through the liquid transmission aperture T1 formed in the seal member 20, so as to flow into a space on the medical agent N1 side. This enables the drug solution M1 to be mixed with the medical agent N1, leading to generation of a mixed fluid M2 formed with the drug solution M1 and the medical agent N1. Note that as illustrated in FIGS. 15 and 16, the mixing operation of the drug solution M1 and the medical agent N1 is performed with the cap 51 attached to the fixed part 7 of the syringe 2. In addition, with operation of pushing and pulling the push rod 11 of the pusher 4, it is possible to efficiently perform the mixing operation of the drug solution M1 and the medical agent N1.

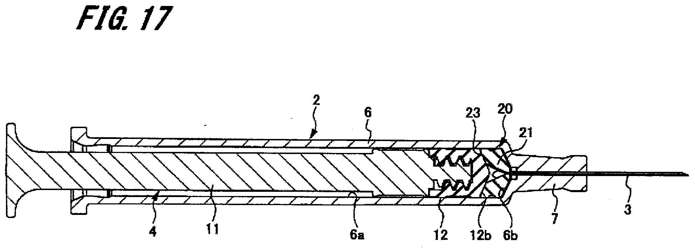

Next, as illustrated in FIG. 17, the cap 51 is removed, and thereafter the user further pushes the push rod 11 of the pusher 4 to further slide and move the gasket 12 toward the inner distal end surface 6b, allowing the seal member 20 to slide and move together with the gasket 12 within the cylinder hole 6a toward the inner distal end surface 6b. As a result, the mixed fluid M2 of the drug solution M1 and the medical agent N1 is pushed out by the seal member 20 and the gasket 12, and is discharged from the needle tube 3. This completes the administration operation of the prefilled syringe 41.

Since other configurations are similar to the configuration of the prefilled syringe 1 according to the first exemplary embodiment, description will be omitted. The above-configured prefilled syringe 41 can also obtain the similar operational effects as the prefilled syringe 1 according to the first exemplary embodiment described above.

The exemplary embodiments of the present invention have been described as above including its operational effects. The seal member and the prefilled syringe according to the present invention are not limited to the above-described embodiments, and various modifications are possible within the scope of the invention described in the claims.

While the above-described exemplary embodiment is an example of providing one seal member 20 alone, the present invention is not limited to this example. For example, a plurality of seal members may be disposed in the cylinder hole of the cylinder body in the syringe, with a plurality of types of drug solutions of different types stored in the syringe.

In addition, while the above is an example in which the needle tube is fixed to a syringe, the present invention is not limited thereto. The needle tube and the syringe may be configured to be detachable from each other and the needle tube may be attached to the syringe when the drug solution is administered.

### Reference Signs List

- 1, 41: Prefilled syringe
- 2: Syringe
- 3: Needle tube
- 3a: Needle point
- 3b: Proximal end part
- 4: Pusher
- 5: Cap
- 5a: Cylinder hole
- 6: Cylinder body
- 6a: Cylinder hole
- 6b: Inner distal end surface
- 7: fixed part (holding part)
- 11: Push rod
- 12: Gasket
- 12a: Coupled part
- 12b: Distal end part
- 20: Seal member
- 21: Main body part
- 21a: Distal end side rib part
- 21b: Proximal end side rib part
- 21c: Separation part
- 22: Closed end part
- 23: Aperture part
- 24: pressurizing part
- 24a: Pressurizing surface
- 25: Cleavable part
- 25a: Pressurized surface

- 25b: Linear part
- M1: Drug solution
- M2: Mixed fluid
- N1: Medical agent
- S1: Slit
- T1: Liquid transmission aperture

## Claims

1. A seal member configured to be used with a syringe assembly comprising a syringe including a cylinder body that is configured to be filled with a drug solution, the seal member comprising:
a tubular main body part including an aperture part formed in a proximal end thereof and a tight contact part configured to form a fluid-tight seal with an inner wall of the cylinder body;
a closed end part integrally and continuously formed with the main body part at a distal end of the main body part to seal the distal end of the main body part; and
a cleavable part formed at the closed end part and connecting with the aperture part,
wherein the main body part is disposed in the cylinder body together with a gasket slidable within the cylinder body, and is positioned distal of the gasket in the cylinder body, so as to form a drug solution storage space to store the drug solution together with the gasket and the cylinder body, and
wherein, with a prescribed pressure applied to the drug solution storage space, the cleavable part is configured to be cleaved and form a liquid transmission aperture connecting with the aperture part at the closed end part.

2. The seal member according to claim 1,
wherein a portion of the closed end part in which the cleavable part is disposed is formed to be thinner than the other portions.

3. The seal member according to claim 1,
wherein the cleavable part comprises two pressurized surfaces to be brought close to each other in a direction toward a distal end of the closed end part.

4. The seal member according to claim 3,
wherein the main body part comprises, between the aperture part and the cleavable part, a tapered pressurizing part having a flow path continuously reduced as approaching the closed end part.

5. The seal member according to claim 4,
wherein the main body part comprises:
a proximal end side rib part that is provided in a ring shape on an outer circumferential surface of the main body part in a vicinity of the aperture part and that function as the tight contact part; and
a separation part that is provided in a ring shape on an outer circumferential surface of the main body part in a vicinity of the pressurizing part and is formed so as to be separated from an inner wall of the cylinder body when the main body part is disposed in the cylinder body.

6. The seal member according to claim 5,
wherein the main body part
further includes a distal end side rib part that is provided in a ring shape on an outer circumferential surface of the main body part distal of the separation part and in a vicinity of the closed end part and that functions as the tight contact part.

7. The seal member according to any one of claims 1 to 6,
wherein the aperture part opens in a shape complementary to a shape of a distal end part of the gasket.

8. The seal member according to any one of claims 1 to 7,
wherein the cleavable part is linearly formed in a direction orthogonal to an axial direction of the cylinder body.

9. The seal member according to any one of claims 1 to 8,
wherein a distal end surface of the closed end part is formed in a shape complementary to a shape of an inner distal end surface of the cylinder body.

10. A syringe assembly comprising;
the seal member according to any one of claims 1 to 9; and
a syringe having a cylinder body configured to be filled with a drug solution,
wherein the seal member is disposed in the cylinder body.

11. The syringe assembly according to claim 10, further comprising a needle tube that includes a needle point configured to puncture a living body,
wherein the syringe includes a holding portion that holds the needle tube.

12. A prefilled syringe comprising:
the syringe assembly according to claim 10 or 11;
a gasket slidably disposed in the cylinder body and positioned proximal of the seal member; and
the drug solution stored in a drug solution storage space formed by the seal member, the gasket, and the cylinder body.

13. The prefilled syringe according to claim 12,
wherein the seal member is slidably disposed within an intermediate part of the cylinder body in an axial direction, and
a medical agent to be mixed with the drug solution is stored in a medical agent storage space formed between the seal member and an inner distal end surface of the cylinder body.
